# EUROPEAN PATENT SPECIFICATION

(11) **EP 3 180 602 B1**
(45) Date of publication and mention of the grant of the patent: **30.09.2026**
(21) Application number: 15832114.1
(22) Date of filing: 12.08.2015
(51) Int. Cl.: G01N 22/04, G01N 33/02

(54) **A MICROWAVE MOISTURE METER AND SENSOR**
MIKROWELLENFEUCHTIGKEITSMESSGERÄT UND -SENSOR
DISPOSITIF DE MESURE ET CAPTEUR D'HUMIDITÉ À HYPERFRÉQUENCES

(30) Priority: 14.08.2014 US 201462037184 P
(43) Date of publication of application: 21.06.2017
(73) Proprietor: Dickey-John Corporation, St. Paul, MN 55126 (US)
(72) Inventor: ITAGI, Amit Vasant, Ellicott City, Maryland 21043 (US)
(74) Representative: Lewis Silkin LLP
(86) International application number: PCT/US2015/044800
(87) International publication number: WO 2016/025569

(56) References cited:
- EP-A2- 0 806 654
- EP-A2- 1 573 305
- EP-B1- 1 573 305
- WO-A1-00/14552
- WO-A1-00/14552
- WO-A2-2009/063497
- KR-A- 20140 082 376
- US-A- 3 818 333
- US-A- 4 319 185
- US-A- 5 333 493
- US-A- 5 333 493
- US-A- 5 621 330
- US-A- 5 621 330
- US-A- 6 147 503
- SANG HA NOH ET AL: "Dielectric Properties of Rice at Frequencies from 50 HZ to 12 GHZ", TRANSACTIONS OF THE AMERICAN SOCIETY OF AGRICULTURAL ENGINEERS., vol. 32, no. 3, 1 January 1989 (1989-01-01), US, pages 0991 - 0998, XP093048463, ISSN: 0001-2351, Retrieved from the Internet <URL:http://dx.doi.org/10.13031/2013.31104> DOI: 10.13031/2013.31104

## Description

### CLAIM OF PRIORITY

### FIELD AND BACKGROUND OF THE INVENTION

The invention generally relates to microwave sensors for detecting moisture content in agricultural materials.

Moisture content of materials is a key parameter in many research and industrial applications, including the food and agriculture-related industries. The most widely used standard techniques for moisture content determination are various forms of oven drying. These techniques are based on drying samples under specific conditions, such as temperature and time, depending on the material. Besides being energy and time consuming, in some instances the representative character of the samples might be questionable compared to the whole volume or mass of material under consideration. Moreover, most industrial processes are highly automated and require real-time, on-line measurement of the moisture content.

For grains, moisture is an important factor affecting price paid for grain. Therefore, moisture content must be determined whenever grain is traded. If moisture content is too high at the time of harvest, the grain kernels can be damaged in the mechanical harvesting process, leaving them more susceptible to infection by fungi. If they are stored at moisture contents too high for the prevailing environment, they can spoil because of the action of microorganisms, and the value is degraded or completely lost for human and animal consumption. Electrical measurement methods have been developed that depend on correlations between the electrical properties of the grain and moisture content and grain moisture meters in the United States today are predominantly those operating at about 149MHz and that sense the dielectric properties (relative permittivity) of the grain samples.

Research on sensing moisture content in grain by microwave measurements has indicated two important advantages for microwave frequencies. The inconsistency of moisture measurements by instruments operating in the low frequency range may be due, in part, to the influence of ionic conduction on the measured dielectric properties at high moisture levels. At microwave frequencies, the influence of ionic conduction is negligible, and better correlations between permittivity and moisture content can be expected.

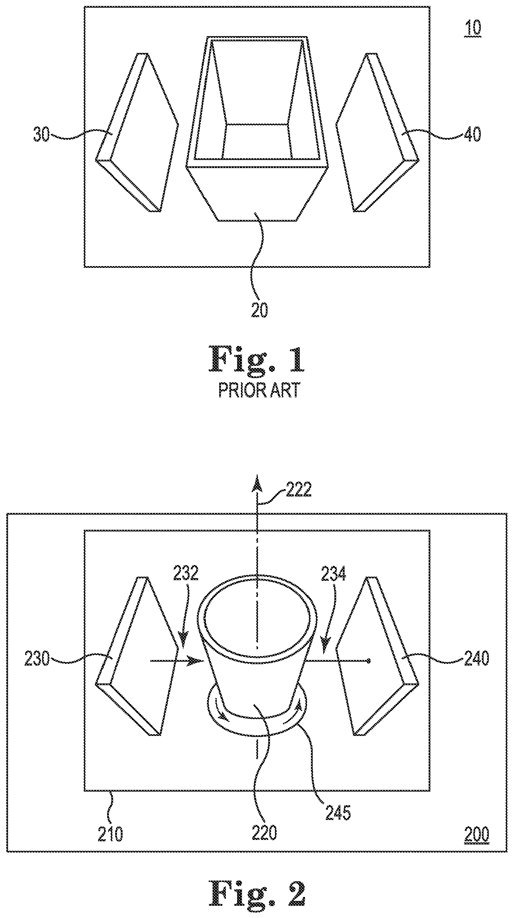

In U.S Patent 8,629,681, Trabelsi et al provide a method that appeared to solve some of the problems associated with the measurement of density and moisture content of bulk materials. Fig. 1 shows part of the basic concept of the microwave measurement system 10 proposed by Trabelsi et al, which includes a rectangular sample holder 20 that is placed between two directional antennas. One antenna acts as a transmit (Tx) antenna 30 and the other acts as a receive (Rx) antenna 40. The phase and attenuation of the microwave beam as it traverses sample holder 20 is measured with and without peanuts in sample holder 20. Trabelsi et al appear to teach that a relative change in the phase and attenuation of the microwave beam that traverses sample holder 20 is used to estimate the moisture content of the peanuts. A correction is then applied to correct for the temperature of the peanut sample.

During the testing of the Trabelsi '681 moisture meter prototype in a grain lab, it was discovered that the moisture meter provided adequate results, however the levels of accuracy and repeatability of results demanded by users in the market are higher. Hence there is a need in the market for a microwave-based moisture meter system exhibiting higher levels of measurement accuracy with consistent repeatability of outcomes.

KR 2014 0082376 discloses a microwave transceiver for measuring a grain moisture content based on the attenuation of microwaves transmitted through a sample holder by using two frequencies of 2.38 GHz of an S-band and 10.5 GHz of an X-band.

### SUMMARY

According to an embodiment of the present invention, there is provided a method of measuring moisture within a material sample by a microwave moisture meter as set out in claim 1. Preferred features are set out in the dependent claims.

### BRIEF DESCRIPTION OF THE DRAWINGS

Other important objects and advantages of the present invention will be apparent from the following detailed description of the invention taken in connection with the accompanying drawings in which:
FIG. 1 is a prior art arrangement for a microwave moisture sensor;
FIG. 2 illustrates a microwave moisture meter configuration in accordance with the invention;
FIG. 3 is a graph that illustrates a comparison of variance contributions from different sources;
FIGS. 4A and 4B are graphs that illustrate a comparison for two moisture meter configurations of attenuation/phase ratios accordance with the invention;
FIG. 5 illustrates another microwave moisture meter configuration in accordance with the invention;
FIG. 6 is a graph that illustrates attenuation/phase ratio for a cylinder with matching block geometry configuration for a moisture meter in accordance with the invention;
FIG. 7 is a graph that illustrates linear polarization in accordance with the invention; and
FIG. 8 is a graph that illustrates circular polarization in accordance with the invention.

### DETAILED DESCRIPTION OF THE INVENTION

Following below are more detailed descriptions of various embodiments of an improved microwave moisture meters or sensors and systems described herein. It should be appreciated that various aspects of the subject matter introduced above and discussed in greater detail below may be implemented in any of numerous ways, as the subject matter is not limited to any particular manner of implementation. Examples of specific implementations and applications are provided primarily for illustrative purposes.

In most of the embodiments described herein, we relied on the microwave moisture meter arrangement (FIG. 1) described in U.S. Patent No. 8,629,681, the disclosure of which is incorporated by reference in its entirety, and focus most of the discussion herein on the sample holder and antenna arrangements and the variations in the moisture meter signal.

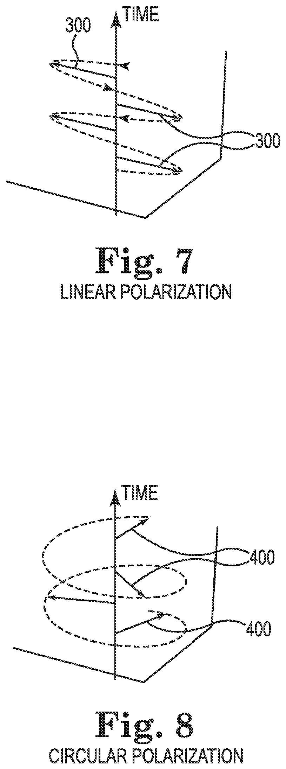

Referring now to the figures, in FIGS. 2 and 7, there is illustrated a sample material holder assembly and antenna configuration 210 of a microwave moisture meter 200 (not shown) that includes a cylindrical sample holder 220 bounded by a transmitting antenna 230 on one side and a receiving antenna 240. In this example embodiment, sample holder 220 includes a longitudinal axis 222 along a length of holder 220 and is rotated axially in either direction by a rotator mechanism, represented by disk or platen 245 (arrows, right circular direction in this example). A polarized microwave signal 232 originates from transmitting antenna 230, passes through holder 220 and the sampled material and is received by receiving antenna 240 as an attenuated wave 234 (which may also exhibit a phase difference as well depending upon the amount of moisture in the sampled material) in holder 220. In this and other example embodiments, platen 245 rotates in either direction, at any speed (since the microwave signals travel so quickly and measurements occur rapidly) and at any speed, which assists in removing variations in measurements that may be caused by the alignment of the individual particles of the sampled material.

In this and most of the following embodiments, the mode of operation will be linear polarization of the microwave propagation, which is illustrated in FIG. 7, in either the vertical or horizontal direction or orientation. With linear propagation, various arrows 300 show the electric field direction at a point in space as a function of time as created by the transmitting antenna, which causes the field to oscillate linearly along a plane normal to the direction of wave propagation. The receiving antenna is reciprocally sensitive to the linear polarization.

In this example embodiment, the microwave frequency used for this measurement is about 5.8 GHz and the measurements are taken using peanuts. The real part of the peanut dielectric constant is around 2, however the exact value changes with moisture (in one example, moisture can range from about 8% to about 20%). In a medium of dielectric constant of about 2, the microwave wavelength is around 3.6 cm. This is comparable to the length/diameter of a peanut pod. Thus, the microwaves do not see the peanuts either as scattering points or as a homogeneous medium. The microwave attenuation/phase therefore will have some variation depending on the physical configuration of the peanuts in the sample holder. Experimentally, it has been seen that this variation is a big contributor to the overall variance of the attenuation and phase measurements in standard rectangular sample holders, which the invention described herein will overcome based on a number of factors.

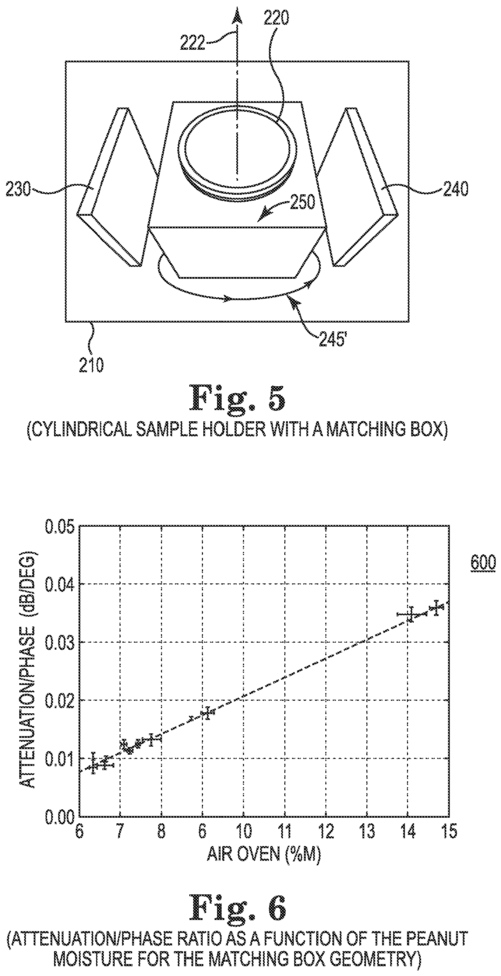

In this example embodiment, the sample holder shape is changed from the rectangular bin in FIG. 1 (prior art) to a cylindrically shaped bin or holder 220. Other shapes for the holder can be used as long as they can be axially rotated during moisture measurement. In a related embodiment, a matching block (see Fig. 5) is included about the cylinder bin. In this example embodiment, sample holder 220 is rotated and the average is taken of the data collected over the different angular positions of sample holder 220.

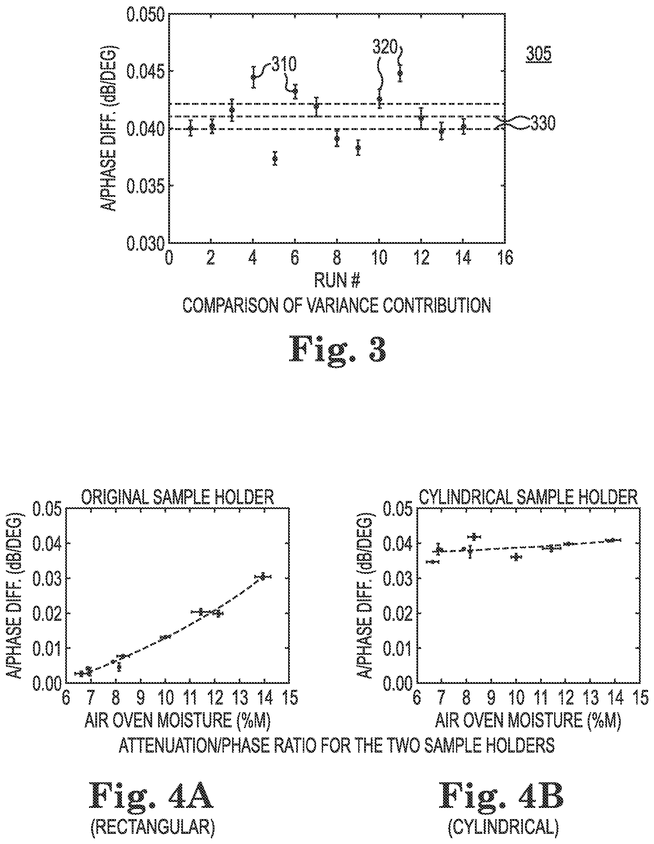

Referring now to Fig. 3, there is shown a graph 305 that illustrates the attenuation/phase ratio for multiple peanut runs/drops, which is basically a comparison of variance contribution to the measurement outcomes. A drop refers to a draw or removal of a peanut sample from the peanut population. Points or dots 310 and the error bars 320 show the average over the angular position and the variation over the angular positions, respectively. The dashed lines 330 represent the variation between points 310 from the different runs. In this plot 305, the error bars represent a 95% confidence interval for the mean. It can be seen that the variation over the angular positions is comparable to the variation over multiple drops. Thus, reducing the first contribution to the variance reduces the total variance considerably.

Although arrangement 210 is an improvement over the prior art, using cylindrical sample holder 220 presents a challenge with respect to some of the measurements. The peanuts have a permittivity of (about) ~ 2. Thus, a cylindrical mass, such as cylindrical holder 220, can act as a cylindrical lens. The "lens" focuses the microwave beam from the transmitting antenna to a smaller size as it reaches the receiving antenna. This causes a loss in sensitivity at receiving antenna 240, which is illustrated FIG. 4B versus the rectangular holder shown in Fig. 1 (Fig. 4A is the rectangular holder). The attenuation/phase ratio is plotted for the two sample holders as a function of the peanut moisture measured with an air oven. The X-axes in Figs. 4A, 4B, and 6 show the air oven moisture, which is the moisture in the sample measured independent of the moisture meter when drying the sampled material in an air oven.

Referring now to Fig. 5, in order to mitigate this loss in sensitivity due to the lensing effect, sample holder (or inner holder member) arrangement 210 is reconfigured so as to use a rectangular block 250 (or any other shape) disposed about and around cylindrical sample holder 220. In this example embodiment, holder 220 is configured to be rotated by a rotator 245' (not shown, only arrow of rotating direction with respect to axis 222) separate from block or outer holder member 250. In this example embodiment, the material of the rectangular block 250 has a permittivity in the range of about 2-3. For the transmitted polarized microwave signals, the matching block appears as an approximate continuation of the cylindrical peanut mass (or whatever material is being measured). The permittivity material matching as well as the rotation of the sample holder prevents the strong lensing (caused by cylindrical holder surface) from occurring. In other embodiments, other materials and shapes can be used for block 250 that approximates the permittivity of the material being measured and achieves improved measurements depending upon the application in which it is applied (peanuts, grains, seeds, coffee beans, etc..). In a related embodiment, a rectangular block similar to block 250 is formed with a cavity of desired shape or a cylindrical hole, thereby dispensing with the cylindrical holder 220 if the user desires. In yet another related embodiment, a cylindrical sleeve with a permittivity/dielectric constant in the same range as the material being measured can also be used around cylindrical holder 220, a range for the permittivity/dielectric constant around 2-10.

Referring now to Fig. 6, there is shown a graph that illustrates the attenuation/phase ratio once the rectangular block 250 is used with cylindrical holder 220. In this example, the Attenuation/Phase ratio is shown as a function of the peanut moisture for the matching block geometry. Note that the sensitivity of the embodiment in Fig. 5 is considerably more than what was seen on Fig. 4B. In addition, the standard deviation in the measurement is around 50-60% of what was measured with the original rectangular sample holder 20.

Referring now to FIG. 8, in a related embodiment, another solution for sensing or detecting moisture is to use circular polarization, with arrows 400 showing the electric field direction at a point in space as a function of time formed by the transmitting and the receiving antennas of the metering arrangement. An advantage to using circular polarized signals is the reduction of variation in the moisture measurements that is caused by the differing orientation of the many particles, in this case peanuts, in the sample material to be measured. In the original design, the antennas are operated with linear polarization (either horizontal or vertical), but they can also be operated in the elliptical polarization mode. There will be a variation coming from the percent alignment of the long axis of the peanuts with the polarization of the electric field. Thus, if two different peanut loading schemes cause the peanut alignment to change, then they could read or detect slightly different levels of moisture. Hence, to address this issue of contradictory measurements of moisture levels, the transmitting and receiving antennas are operated with the circular polarization configuration shown in FIG. 8. This causes the polarization of the microwave to rotate in a circle in a plane normal to the direction of propagation, hence traveling in a corkscrew fashion from the transmitting antenna through the sampled material to the receiving antenna and providing more complete coverage over the entire sampled material. Thus, combining this approach with the cylinder shaped sample holder and the rotation approach of FIG.2 and FIG. 5, the fraction of the time that the electric field aligns with a peanut will approximately be the same for every peanut (or any other grain or kernel used with this approach), thereby eliminating the contradictory moisture measurements.

### Appendix A

### SUMMARY OF THE INVENTION (U.S. Patent No. 8,629,681)

It is therefore an object of the present invention to provide a microwave sensor for instantaneous and nondestructive determination of bulk density and moisture content in a granular or particulate material measured at a single microwave frequency.

A still further object of the present invention is to provide a microwave circuit for use with a microwave sensor which operates at a single microwave frequency and utilizes a novel algorithm for instantaneous and nondestructive determination of bulk density and moisture content in a granular or particulate material.

A still further object of the present invention is to provide a microwave circuit utilizing a novel algorithm for correctly calculating real and imaginary parts of complex permittivity from measurements of attenuation and phase shift of a wave traversing a granular or particulate material.

A still further object of the present invention is to provide a microwave sensor for instantaneous and nondestructive determination of bulk density and moisture content in a granular or particulate material wherein said sensor includes a microwave source, an isolator, a power splitter, a transmitting antenna, a receiving antenna, a temperature measurement device, a mixer, an analog-to-digital converter, a data acquisition and computing unit, and a sensor display.

Another object of the present invention is to provide a means for determining the real part and the imaginary part of the relative complex permittivity from single-frequency measurement of the attenuation and phase shift of a microwave signal after it traverses a sample material.

A still further object of the present invention is to provide a novel algorithm for phase correction, based on a linear relationship between the attenuation per unit thickness and the dielectric constant to resolve phase ambiguity for samples with thickness greater than the wavelength in the material.

Further objects and advantages of the present invention will be apparent from the following detailed description.

### BRIEF DESCRIPTION OF THE DRAWINGS

FIG. 1 is a drawing of a microwave sensor including a microwave source, an isolator, a power splitter, a transmitting antenna, a receiving antenna, a temperature measurement device, a mixer, an analog-to-digital converter, a data acquisition and computing unit, and a sensor display.
FIG. 2 is a graph showing the variation of attenuation divided by sample thickness as a function of dielectric constant of peanut kernels where f=approximately 5.8 GHz.
FIG. 3 is a graph showing the variation of dielectric loss factor divided by bulk density as a function of dielectric constant divided by bulk density for peanut kernels at f=approximately 5.8 GHz.
FIG. 4 is a graph showing the variation of a density-independent calibration function with moisture content at approximately 5.8 GHz and approximately 23 degrees C.
FIG. 5 is a graph showing the variation of a density-independent calibration function .psi..sub.k with moisture content M.sub.k and temperature T.
FIG. 6 is a graph showing the variation of a density-independent calibration function .psi.sub.p with moisture content M.sub.p and temperature T.
FIG. 7 is a photograph of the microwave sensor without an upper cabinet.
FIG. 8 is a photograph of the microwave sensor and sample holder bucket of the present invention wherein the sample holder bucket is filled with peanut pods.
FIG. 9 is a flow diagram for the phase correction algorithm.

### DETAILED DESCRIPTION OF THE INVENTION

The present invention is a microwave sensor (FIG. 1), that includes a microwave circuit, operating at a single frequency for instantaneous and nondestructive determination of bulk density and moisture content of a granular or particulate material, such as for example, peanuts, grains, seeds, biofuel pellets, and other granular and particulate materials. The microwave circuit provides means for determining the real part and the imaginary part of the relative complex permittivity from measurement of the attenuation and phase shift of a microwave signal after it traverses the sample material under test. The circuit further includes a novel algorithm for phase correction (FIG. 9), based on a linear relationship between the attenuation per unit thickness and the dielectric constant and is used to resolve the phase ambiguity for samples with thickness greater than the wavelength in the material. The dielectric constant and dielectric loss factor are calculated from measurements of the attenuation and phase shift. Once the dielectric properties are determined, the bulk density of the material is calculated based on a complex-plane representation of the dielectric properties, and moisture content is determined with a density-independent calibration function expressed in terms of the dielectric properties. Also described is a method for moisture determination in a material using the microwave circuit of the present invention and measurements of dielectric properties.

Dielectric properties of materials are intrinsic electrical properties that characterize the interaction between the electric field of an electromagnetic wave and the material. They are represented by the relative complex permittivity .di-elect cons.=.di-elect cons.'-j.di-elect cons.", where .di-elect cons.' is the dielectric constant, .di-elect cons." is the dielectric loss factor, and j= {square root over (-1)} is the imaginary unit. Both .di-elect cons.' and .di-elect cons." are dependent on frequency, temperature, water content and composition of the material (Hasted, 1973, Nelson, 1981). At microwave frequencies, dielectric properties have been used for nondestructive simultaneous determination of moisture content and bulk density of granular and particulate materials (Trabelsi et al., 2000b. Method for the simultaneous and independent determination of moisture content and density of particulate materials from radio-frequency permittivity measurements, USA, U.S. Pat. No. 6,147,503; Trabelsi et al., 2004, Universal dielectric calibration method and apparatus for moisture content determination in particulate and granular materials, USA, U.S. Pat. No. 6,691,563). Accuracy and repeatability of moisture content and bulk density predictions rely mainly on the accuracy with which .di-elect cons.' and .di-elect cons." are measured and the stability of the calibration equations correlating these properties with moisture content and bulk density. For accurate measurement of .di-elect cons.' and .di-elect cons.", sophisticated and expensive instruments such as impedance and vector network analyzers are often used. It is the object of this invention to provide an inexpensive microwave sensor operating at a single microwave frequency and associated algorithm for accurate determination of .di-elect cons.' and .di-elect cons." that are used to determine moisture content and bulk density of the material.

### Microwave Sensor

Many techniques can be used for measurement of the dielectric properties of a given material (Bussey, 1967. Measurement of the RF properties of materials, a survey. Proc. IEEE, 55(6), 1046-1053; Von Hippel, 1954. Dielectrics and Waves. John Wiley & Sons, New York). Among these, free-space techniques (reflection and transmission) have the advantages of being nondestructive, not requiring any sample preparation, and not requiring any physical contact with the material (Nyfors and Vainikainen, 1989. Industrial Microwave Systems. Artech House, Norwood, Mass., 351 pp.). The microwave sensor of the present invention is based on a free-space transmission technique in which .di-elect cons.' and .di-elect cons." are determined from measurement of the attenuation and phase shift the incident wave undergoes when propagating through the material. FIG. 1 shows the diagram of the microwave sensor which is composed of the following elements:
1--Microwave source
2--Isolator
3--Power splitter
4--Transmitting antenna
5--Receiving antenna
6--Temperature measurement device
7--Mixer
8--Analog-to-digital converter
9--Data acquisition and computing unit
10--Sensor display
11--Sample

The microwave source 1 is connected to an isolator 2 which will prevent any reflected signal from reaching the source 1. The isolator 2 is connected to a power splitter 3 with two output ports 1 and 2. The microwave signal at port 1 is the reference signal and is sent to the LO port of the mixer 7. The microwave signal at port 2 is sent to the transmitting antenna 4 which in turn radiates the electromagnetic energy into the material sample under test. Facing the transmitting antenna 4 is the receiving antenna 5 which collects the radiation after it traverses the sample 11 and sends it to the RF port of the mixer 7. In the mixer 7, the RF input is divided into two paths, in one path the RF input is mixed with the LO signal and provides the in-phase (I) signal, in the second path the RF signal is mixed with the 90-degree phase-shifted LO signal and provides the quadrature (Q) signal. Since both the LO and RF signals are at the same frequency, the voltages at the mixer outputs have dc values and are expressed as follows: V.sub.1=V.sub.RFK cos .theta. (1) V.sub.Q=V.sub.RFK sin .theta. (2)

.theta..function. ##EQU00001## where V.sub.RF is the instantaneous peak RF voltage, K is the conversion loss of the mixer, and .theta. is the phase angle between the RF and LO signals. Both voltages V.sub.1 and V.sub.Q and temperature T are fed to the data acquisition and computing unit 9 where .di-elect cons.' and .di-elect cons. are calculated and moisture content and bulk density are determined. This will be explained in the following. Frequency and Sample Dimensions Selection

The frequency is selected to avoid effects of ionic conduction which affect negatively the accuracy and repeatability of moisture content measurements (Trabelsi et al., 1998. New density-independent calibration function for microwave sensing of moisture content in particulate materials. IEEE Transactions on Instrumentation and Measurement, 47(3), 613-622). It is well established that the effect of ionic conduction is negligible at frequencies higher than 3 GHz (Hasted, 1973. Aqueous Dielectrics. Chapman and Hall, London). Also, to avoid electromagnetic wave scattering by the material, the wavelength of the incident wave should be larger than the size of granules or particles constituting the material.

Accuracy of attenuation measurements is affected by diffraction at the edges of the sample 11, multiple reflections, and scattering by the granules or particles. Diffraction at the edges of the sample can be minimized by selecting the transverse dimensions of the sample about three times the E-plane 3 dB beamwidth of the antenna (Chen et al., 2004. Microwave electronics. Wiley, West Sussex, England, 537 pp). Multiple reflections occur inside the sample, between the sample interfaces and the antennas and between the antennas through the sample. Effect of multiple reflections can be minimized by selecting a sample thickness (d) that ensures a minimum of 8 to 10 dB one-way attenuation and selecting the best matching condition between the two antennas 4 and 5. It was also observed that a minimum of a one-wavelength distance between each antenna 4 and 5 and the sample 11 is required. Finally, effects of scattering by the granules or particles can be reduced by proper selection of the frequency and material thickness (Trabelsi et al., 1999. Determining physical properties of grain by microwave permittivity measurements, Transactions of the ASAE, 42(2), 531-536; Trabelsi and Nelson, 2006. Nondestructive sensing of physical properties of granular materials by microwave permittivity measurement, IEEE Transactions on Instrumentation and Measurement, 55(3), 953-963).

### Attenuation and Phase-Shift Determination

Attenuation and phase shift are determined by comparing the reference microwave signals measured without the sample to those obtained after the sample 11 is placed between the two antennas 4 and 5.

Attenuation is the difference between the power levels without the sample (P.sub.RF,0) and with sample (P.sub.RF,S) placed between the transmitting and receiving antennas 4 and 5. The power level at the RF port is calculated as:
Equation (not shown) where V.sub.RF is:
Equation (not shown) And R is the input resistance to each device, which is 50.OMEGA. for a 50-.OMEGA.-based system. The power level at the RF port can be expressed in dB as:
Equation (not shown) The attenuation in dB is calculated from equations (4) through (6) (not shown) as: .DELTA.A(dB)=P.sub.RF,S(dB)-P.sub.RF,0(dB) (7)
Phase shift characterizes the delay in propagation caused by the slowing of speed of propagation of the wave in the medium. The phase shift is calculated as the difference between the phase measured without the sample 11 (.theta..sub.0) and with sample 11 (.theta..sub.S) placed between the transmitting and receiving antennas 4 and 5. The phase shift is calculated by using equations (1) through (3) as: .DELTA..theta.(rad)=.theta..sub.S-.theta..sub.0 (8) Because of the requirements cited above for accurate measurement of the attenuation, very often the sample 11 thickness is greater than the wavelength in the medium. Therefore, an ambiguity in phase determination occurs and there is need for a phase correction.

Therefore, the actual phase shift is expressed as:
.DELTA..theta..sub.actual(rad)=(.theta..sub.S-.theta..sub.0)-2.pi.n (9) where n is an integer to be determined. Solutions for resolving the phase ambiguity have been proposed (Musil and Zacek, 1986. Microwave Measurement of Complex Permittivity by Free Space Methods and Applications, Elsevier, New York, N.Y.; Trabelsi et al., 2000c. Phase-Shift ambiguity in microwave dielectric properties measurements, IEEE Transactions on Instrumentation and Measurement, 49(1), 56-60). However, they either require measurements at two frequencies or impose restrictions on the choice of the sample 11 thickness. A novel and original solution for the phase ambiguity problem will be described below. Computation of the Dielectric Properties

The dielectric properties, real and imaginary parts, respectively, of the relative complex permittivity, .di-elect cons.=.di-elect cons.'-j.di-elect cons.", are calculated from measurement of the attenuation and phase using equations (not shown) where.lamda..sub.0 is the free-space wavelength, c is the speed of light in vacuum in m/s and f is the frequency in Hertz. Both .DELTA.A and .DELTA..theta..sub.actual are taken as positive numbers. Algorithm for Phase Correction

When the thickness of the sample 11 is greater than the wavelength in the sample 11, an ambiguity in the phase determination occurs and therefore the phase needs to be corrected for the computation of the dielectric properties of the sample 11. Available methods for phase correction require either measurements at two frequencies or measurements with two different thicknesses. There is no method for phase correction for measurements of dielectric properties at a single frequency and single sample 11 thickness. A novel phase correction algorithm of the present invention for phase correction for measurements of the dielectric properties at a single frequency without restrictions on the sample 11 thickness is disclosed (FIG. 9). The new phase correction algorithm uses the attenuation as an indicator to determine the expected range of .di-elect cons.' and consequently the proper value of n to be used in equation 9. Once n is determined, the actual phase is calculated with equation 9 and the dielectric properties are computed from the attenuation (equation 7) and phase shift (equation 9) with equations 10 and 11 and subsequent set of equations 12-15. For this purpose, for a given sample 11, as a first step, the plot of attenuation divided by the material thickness (.DELTA.A/d) versus the dielectric constant (.di-elect cons.') is obtained (FIG. 2). A table is generated from this plot, whereby for each range of attenuation divided by thickness a corresponding range for .di-elect cons.' is identified. From this table, for each measured value of .DELTA.A/d, the expected range for .di-elect cons.' is determined. The phase ambiguity is resolved by looking for a value of n for which the calculated .di-elect cons.' falls within that range. Note that none of the physical properties of the sample 11 including bulk density, moisture content, and temperature is needed in the phase correction algorithm.

Rather than starting the search for n (equation 9) with a random guess, the measured value of attenuation divided by the sample thickness is used to identify the range of expected dielectric constant s'. For purpose of illustration, refer to FIG. 2. Let's say the measured value of .DELTA.A/d is between 0.5 and 1 dB/cm, then from FIG. 2, the expected range for .di-elect cons.' is from 2.25 to 2.6. Starting with n=1 in equation (9), the dielectric constant .di-elect cons.' is calculated with equation (10) and equations (12) through (15). If the value obtained for .di-elect cons.' is within the range from 2.25 to 2.6, then n=1 is the solution. If the value obtained for .di-elect cons.' is not within that range, then n is incremented by 1, and a new value of .di-elect cons.' is calculated following the same procedure used for n=1. This process continues until a value of .di-elect cons.' within the range from 2.25 to 2.6 is found. Once the phase ambiguity is resolved, .di-elect cons.'s is calculated with equation (11) and equations (12) through (15).

### Simultaneous and Independent Determination of Bulk Density and Moisture Content

Once .di-elect cons.' and .di-elect cons." are determined, a method covered in U.S. Pat. No. 6,147,503, issued Nov. 14, 2000 (Trabelsi et al., 2000b); herein incorporated by reference in its entirety, is used to determine moisture content and bulk density from measurement of the dielectric properties at a single microwave frequency.

Bulk density is determined from a complex-plane representation of the dielectric properties .di-elect cons.' and .di-elect cons." each divided by density as shown in FIG. 3.

To correlate dielectric loss factor divided by bulk density with dielectric constant divided by bulk density, a linear regression (not shown) is used.

For the results presented in FIG. 3, the slope a.sub.f was 0.577, the .di-elect cons.'/p-axis intercept k was 3.1, and the coefficient of determination r.sup.2=0.98.

The bulk density is calculated from equation (16) (not shown) without knowledge of the moisture content and temperature of the material.

Moisture content is determined with a density-independent calibration function (Trabelsi et al., 2000a) expressed in terms of .di-elect cons.' and .di-elect cons." as:
Equation (not shown) where a.sub.f is the regression-line slope determined from the complex-plane representation of the dielectric properties, each divided by bulk density, at a given frequency. For peanut kernels, a.sub.f was 0.577. The relationship between .psi. and moisture content is illustrated in FIG. 4.

A linear regression provides the relationship between .psi. and moisture content M: .psi.=0.0233M+0.056 (19) The coefficient of determination for equation (19) was 0.99. The moisture calibration equation can be obtained from an equation (19).

The microwave sensor 20 of the present is housed in an aluminum cabinet of rectangular cross-section composed of two parts, a base cabinet and a cabinet cover. The base cabinet, which is a closed rectangular box about 18 inches wide, 12 inches deep, and 5 inches high, houses the microwave circuit components, fused power switch, power supply, cooling fan, and analog-to-digital interface, and serves as a mounting platform for the transmitting and receiving antennas, which face each other in alignment with L-shaped mounting brackets spaced 111/2 inches apart above the base cabinet (FIG. 7). The cabinet cover consists of a four-sided rectangular aluminum box, 18 inches wide, 12 inches deep and 10 inches high with an open bottom, which rests on the base cabinet with end plates overlapping the base cabinet to facilitate mechanical attachment. The top of the cabinet cover consists of a 1/2-inch polyvinylchloride (PVC) plate with a 51/4 by 83/4 inch opening to accept a sample holding bucket, which is lowered into the chamber between the two antennas to place the sample in position between the antennas for the measurement (FIG. 8). When assembled to the cabinet base, the cabinet cover encloses the antennas and, with the top of the base cabinet, forms the antenna chamber for the microwave sensor. Sample holding buckets (FIG. 8) were fabricated from 1/4-inch polycarbonate (Lexan) plate. They are a rectangular cross-section bucket with outside dimensions 51/4 inches wide, 83/4 inches deep and 85/8 inches high with a 1/2-inch wide flange at the top that rests on a recessed lip of the opening in the PVC top of the cabinet to support and register the sample holding bucket in position between the two antennas. The Lexan buckets were assembled with cement and Nylon screws to avoid reflections of microwaves from metallic objects. The entire interior of the antenna chamber was lined with microwave absorbing material to prevent unwanted reflections that interfere with the desired measurements. Microwave circuit components were assembled and mounted on an aluminum plate suspended below the top of the base cabinet with aluminum posts. Coaxial cables connect the antennas through the top of the base cabinet to the microwave circuits housed in the base cabinet. A removable plate, forming the bottom of the base cabinet, was provided for assembly of the components and access for servicing the instrument. A filtered, circulating fan was mounted in one end of the base cabinet to provide cooling and ventilation for the components. The analog-to-digital converter was also mounted in the base cabinet with its cable connection socket available on the rear panel of the base cabinet for connection to a computer or microprocessor.

The following example is presented to illustrate the use of the present invention for moisture and density determination of a material sample. Peanuts are used as a test model in the present invention. The example is intended to illustrate the invention and is not intended to limit the scope of the defined claims.

### Example 1

During the grading process, peanut pod samples are shelled and cleaned to determine the moisture content in the peanut kernels. This is somewhat tedious and time consuming. A method is disclosed for determining moisture content in peanut kernels from measurement of the dielectric properties of samples of clean or unclean peanut pods without having to shell them. The method only requires the introduction of pod samples to the sensor for an instantaneous determination of moisture content. Calibration of the sensor prior to use consists of collecting dielectric properties data for the peanut kernel and peanut pod samples of different moisture contents, bulk densities, and temperatures at a single microwave frequency. Then, the value of the density-independent calibration function given in equation (18) is calculated for each sample following the steps mentioned above. FIGS. 5 and 6 show the variations of .psi..sub.k, for the kernels, and .psi..sub.p, for the unshelled pods, with moisture content and temperature. For both materials, the data fall within a plane for which the equation can be obtained from a 3-D regression. The following equations were obtained:
For peanut kernels: .psi..sub.k=0.028M.sub.k+0.0025T-0.048r.sup.2=0.96 (21) For peanut pods: .psi..sub.p=0.027M.sub.p+0.003T-0.092r.sup.2=0.97 (22) By equating .psi..sub.k and .psi..sub.p, a calibration equation for determining moisture content in peanut kernels from measurement on peanut pods is obtained using another equation (not shown):
Those skilled in the art will recognize that this invention may be embodied in other species than illustrated without departing from the scope of the invention as defined by the appended claims.

## Claims

1. A method of measuring moisture within a material sample by a microwave moisture meter comprising:
providing the material sample having a known permittivity within a cavity of a holder member (220) of a material sample holder assembly, the holder assembly also including an outer holder member (250) disposed outside and about the holder member, wherein the outer holder member is comprised of a material that approximates the permittivity of the sample material being measured within the holder member;
rotating the holder member about a longitudinal axis of the cavity;
transmitting a polarized microwave signal from a transmitting antenna (230) located on a side of the holder assembly toward the holder assembly; and
receiving the polarized microwave signal at a receiving antenna (240) located on a side of the holder assembly directly opposite the transmitting antenna such that the holder assembly is located in between both antennas, the receiving antenna configured to receive the microwave signal transmitted through the outer holder member, the holder member and the material sample, and
measuring any attenuation and/or phase change of the polarized microwave signal.

2. The method of claim 1, wherein the holder member is configured as a substantially cylindrical holder member capable of rotating axially within the outer holder member.

3. The method of claim 1, wherein
the transmitting antenna is configured to transmit the polarized microwave signal in at least one of a circular, linear and elliptical polarization mode to reduce differences in moisture measurements due to orientation of a particle in the sampled material located in the sample holder assembly.

4. The method of claim 3, wherein a rotator mechanism is in operative contact with and configured to axially rotate the holder member within holder assembly.

5. The method of claim 3, wherein a moisture content is detected in the material sample and the permittivity of the outer holder is selected to be in a midrange of a percent moisture content of the individual particles of the material sample.

6. The method of claim 4, wherein a moisture content is detected in the material sample, wherein the holder member is substantially cylindrical in shape and having an oval or elliptical cross section, and wherein the holder member is comprised of material having a permittivity selected to be in a midrange of a percent moisture content of the individual particles of the material sample.

7. The method of claim 1, wherein the mode of polarization of the microwave signal is circular.

8. The method of claim 2, wherein the cylindrically shaped holder member and the antennas are substantially similar in height.

9. The method of claim 2, wherein the outer holder is configured substantially in the shape of a block configured to receive and support the cylindrically shaped holder member between the transmitting and receiving antennas.

10. The method of claim 1, wherein the mode of polarization is linear in either the horizontal or vertical direction.

11. The method of claim 1, wherein the mode of polarization is elliptical.

12. The method of claim 3, wherein a moisture content is detected in the material sample and the permittivity of the holder member and outer holder members is selected to be in a midrange of a percent moisture content of the individual particles of the material sample.

## Patentansprüche

1. Verfahren zum Messen von Feuchtigkeit innerhalb einer Materialprobe durch ein Mikrowellenfeuchtigkeitsmessgerät, umfassend:
Bereitstellen der Materialprobe mit einer bekannten Permittivität innerhalb eines Hohlraums eines Halterelements (220) einer Materialprobenhalter-Anordnung, wobei die Materialprobenhalter-Anordnung weiterhin ein äußeres Halterelement (250) aufweist, das außerhalb und um das Halterelement angeordnet ist, wobei das äußere Halterelement aus einem Material besteht, das der Permittivität des innerhalb des Halterelements gemessenen Probenmaterials annähernd entspricht;
Rotieren des Halterelements um eine Längsachse des Hohlraums;
Senden eines polarisierten Mikrowellensignals von einer Sendeantenne (230), die an einer Seite der Materialprobenhalter-Anordnung angeordnet ist, in Richtung der Materialprobenhalter-Anordnung; und
Empfangen des polarisierten Mikrowellensignals an einer Empfangsantenne (240), die an einer Seite der Materialprobenhalter-Anordnung angeordnet ist, welche der Sendeantenne direkt gegenüberliegt, so dass die Materialprobenhalter-Anordnung zwischen beiden Antennen angeordnet ist, wobei die Empfangsantenne dazu eingerichtet ist, das durch das äußere Halterelement, das Halterelement und die Materialprobe gesendete Mikrowellensignal zu empfangen, und
Messen einer etwaigen Dämpfung und/oder Phasenänderung des polarisierten Mikrowellensignals.

2. Verfahren nach Anspruch 1, wobei das Halterelement dazu eingerichtet ist, ein im Wesentlichen zylindrisches Halterelement zu sein, das axial innerhalb des äußeren Halterelements rotieren kann.

3. Verfahren nach Anspruch 1, wobei die Sendeantenne dazu eingerichtet ist, das polarisierte Mikrowellensignal in mindestens einer von einer zirkularen, einer linearen und einer elliptischen Polarisationsmode zu senden, um Unterschiede in Feuchtigkeitsmessungen aufgrund einer Orientierung eines Partikels in dem in als Probe dienenden Material, das in der Materialprobenhalter-Anordnung angeordnet ist, zu reduzieren.

4. Verfahren nach Anspruch 3, wobei ein Rotationsmechanismus in Wirkkontakt mit dem Halterelement ist und dazu eingerichtet ist, das Halterelement innerhalb der Materialprobenhalter-Anordnung axial zu rotieren.

5. Verfahren nach Anspruch 3, wobei ein Feuchtigkeitsgehalt in der Materialprobe detektiert wird und die Permittivität des äußeren Halters dergestalt gewählt ist, dass sie in einem mittleren Bereich eines prozentualen Feuchtigkeitsgehalts der einzelnen Partikel der Materialprobe liegt.

6. Verfahren nach Anspruch 4, wobei ein Feuchtigkeitsgehalt in der Materialprobe detektiert wird, wobei das Halterelement eine im Wesentlichen zylindrische Form aufweist und einen ovalen oder elliptischen Querschnitt aufweist, und wobei das Halterelement aus einem Material besteht, das eine Permittivität aufweist, die dergestalt gewählt ist, dass sie in einem mittleren Bereich eines prozentualen Feuchtigkeitsgehalts der einzelnen Partikel der Materialprobe liegt.

7. Verfahren nach Anspruch 1, wobei die Polarisationsmode des Mikrowellensignals zirkular ist.

8. Verfahren nach Anspruch 2, wobei das zylindrisch geformte Halterelement und die Antennen im Wesentlichen gleich hoch sind.

9. Verfahren nach Anspruch 2, wobei das äußere Halterelement im Wesentlichen in der Form eines Blocks ausgeführt ist, der dazu eingerichtet ist, das zylindrisch geformte Halterelement zwischen der Sende- und der Empfangsantenne aufzunehmen und zu stützen.

10. Verfahren nach Anspruch 1, wobei die Polarisationsmode in entweder horizontaler oder vertikaler Richtung linear ist.

11. Verfahren nach Anspruch 1, wobei die Polarisationsmode elliptisch ist.

12. Verfahren nach Anspruch 3, wobei ein Feuchtigkeitsgehalt in der Materialprobe detektiert wird und die Permittivität des Halterelements und der äußeren Halterelemente dergestalt gewählt ist, dass sie in einem mittleren Bereich eines prozentualen Feuchtigkeitsgehalts der einzelnen Partikel der Materialprobe liegt.

## Revendications

1. Procédé de mesure de l'humidité à l'intérieur d'un échantillon de matériau au moyen d'un humidimètre à micro-ondes, comprenant les étapes consistant à :
placer l'échantillon de matériau, présentant une permittivité connue, dans une cavité d'un élément de support (220) d'un ensemble de support d'échantillon, l'ensemble de support comprenant également un élément de support externe (250) disposé à l'extérieur et autour de l'élément de support, dans lequel l'élément de support externe est constitué d'un matériau dont la permittivité est proche de celle du matériau d'échantillon mesuré à l'intérieur de l'élément de support ;
faire tourner l'élément de support autour d'un axe longitudinal de la cavité ;
émettre un signal micro-ondes polarisé depuis une antenne émettrice (230) située sur un côté de l'ensemble de support en direction de l'ensemble de support ; et
recevoir le signal micro-ondes polarisé au niveau d'une antenne réceptrice (240) située sur un côté de l'ensemble de support directement opposé à l'antenne émettrice, de telle sorte que l'ensemble de support soit situé entre les deux antennes, l'antenne réceptrice étant configurée pour recevoir le signal micro-ondes transmis à travers l'élément de support externe, l'élément de support et l'échantillon de matériau ; et
mesurer toute atténuation et/ou tout variation de phase du signal micro-ondes polarisé.

2. Procédé selon la revendication 1, dans lequel l'élément de support est configuré sous la forme d'un élément de support sensiblement cylindrique capable de tourner axialement à l'intérieur de l'élément de support externe.

3. Procédé selon la revendication 1, dans lequel l'antenne émettrice est configurée pour émettre le signal micro-ondes polarisé selon au moins un d'un mode de polarisation circulaire, linéaire ou elliptique afin de réduire les différences de mesure d'humidité dues à l'orientation d'une particule du matériau échantillonné situé dans l'ensemble de support d'échantillon.

4. Procédé selon la revendication 3, dans lequel un mécanisme de rotation est en contact fonctionnel avec l'élément de support et configuré pour faire tourner axialement celui-ci à l'intérieur de l'ensemble de support.

5. Procédé selon la revendication 3, dans lequel une teneur en humidité est détectée dans l'échantillon de matériau et la permittivité du support externe est choisie de manière à se situer dans une plage intermédiaire correspondant à un pourcentage de teneur en humidité des particules individuelles de l'échantillon de matériau.

6. Procédé selon la revendication 4, dans lequel une teneur en humidité est détectée dans l'échantillon de matériau, dans lequel l'élément de support présente une forme sensiblement cylindrique avec une section transversale ovale ou elliptique et dans lequel l'élément de support est constitué d'un matériau dont la permittivité est choisie de manière à se situer dans une plage intermédiaire correspondant à un pourcentage de teneur en humidité des particules individuelles de l'échantillon de matériau.

7. Procédé selon la revendication 1, dans lequel le mode de polarisation du signal hyperfréquence est circulaire.

8. Procédé selon la revendication 2, dans lequel l'élément de support de forme cylindrique et les antennes présentent sensiblement la même hauteur.

9. Procédé selon la revendication 2, dans lequel le support extérieur est configuré sensiblement sous la forme d'un bloc configuré pour recevoir et soutenir l'élément de support de forme cylindrique entre les antennes d'émission et de réception.

10. Procédé selon la revendication 1, dans lequel le mode de polarisation est linéaire, soit dans la direction horizontale, soit dans la direction verticale.

11. Procédé selon la revendication 1, dans lequel le mode de polarisation est elliptique.

12. Procédé selon la revendication 3, dans lequel une teneur en humidité est détectée dans l'échantillon de matériau et la permittivité de l'élément de support et des éléments de support extérieurs est choisie de manière à se situer dans une plage intermédiaire d'un pourcentage de teneur en humidité des particules individuelles de l'échantillon de matériau.
